# FASCICULE DE BREVET EUROPEEN

(11) **EP 0 974 333 B1**
(45) Date de publication et mention de la délivrance du brevet: **02.04.2003**
(21) Numéro de dépôt: 99401434.8
(22) Date de dépôt: 11.06.1999
(51) Int. Cl.: A61K 7/00, A61K 7/48

(54) **Composition cosmétique et/ou dermatologique biphasée, utile notamment pour le démaquillage des yeux**
Kosmetische und/oder dermatologische biphasische Zusammensetzung zum Abschminken der Augen
Cosmetic and/or dermatological biphasic composition for eyes make-up removal

(30) Priorité: 01.07.1998 FR 9808417
(43) Date de publication de la demande: 26.01.2000
(73) Titulaire: L'OREAL, 75008 Paris (FR)
(72) Inventeur: Picard, Elisabeth, 78140 Velizy (FR); Marion, Catherine, 92330 Sceaux (FR)
(74) Mandataire: Rasson, Catherine

(56) Documents cités:
- EP-A- 0 370 856
- EP-A- 0 603 080
- WO-A-86/02001
- WO-A-96/14046
- FR-A- 2 655 265

## Description

L'invention a pour objet une composition cosmétique et/ou dermatologique constituée d'une phase aqueuse et d'une phase huileuse distinctes, contenant comme conservateur, du chlorhydrate de poly-hexaméthylène biguanide, et l'utilisation de cette composition pour le nettoyage et/ou le démaquillage de la peau, des muqueuses et/ou des yeux, et notamment pour le démaquillage des yeux sensibles.

Il est connu d'utiliser pour le démaquillage des yeux, des compositions se présentant sous forme de deux phases distinctes qui s'émulsionnent par agitation et se démixent au repos. Une telle composition est par exemple décrite dans le document EP-A-370856.

Ces compositions sous forme biphasée contiennent des conservateurs, notamment des chlorures d'ammonium quaternaire tels que le chlorure de benzalkonium. Or, ces composés entraînent parfois des problèmes de tolérance, notamment pour les sujets ayant les yeux sensibles, et de ce fait on cherche à remplacer ces conservateurs par d'autres qui soient mieux tolérés.

En outre, les conservateurs habituellement utilisés dans les compositions se présentant sous forme d'une seule phase sont souvent peu compatibles avec la forme galénique biphasée. En effet, ces conservateurs traditionnels conduisent à des instabilités physico-chimiques donnant lieu à la formation d'un voile ou d'un précipité dans la phase aqueuse ou bien ils perturbent fortement l'interface eau/huile conduisant à un aspect rédhibitoire de la composition biphasée.

Il subsiste donc le besoin de disposer d'une composition biphasée se conservant bien, sans avoir les inconvénients de l'art antérieur, c'est-à-dire tout en ayant un aspect agréable et une bonne stabilité.

Or, la demanderesse a constaté avec étonnement que le chlorhydrate de poly-hexaméthylène biguanide (nom CTFA : polyaminopropyl biguanide) permettait d'obtenir des compositions biphasées stables ayant de bonnes propriétés de conservation aussi bien physico-chimique que microbienne, tout en permettant un bon démaquillage dans des conditions de confort et de fraîcheur très satisfaisantes.

Rien ne laissait supposer que ce conservateur était susceptible d'être utilisé avec succès dans les compositions biphasées.

La présente invention a donc pour objet une composition cosmétique et/ou dermatologique biphasée constituée d'une phase aqueuse et d'une phase huileuse distinctes, caractérisée en ce qu'elle contient du chlorhydrate de poly-hexaméthylène biguanide.

Le chlorhydrate de poly-hexaméthylène biguanide est présent dans la composition de l'invention en une quantité suffisante pour agir en tant que conservateur dans la composition. Ainsi, ce composé est présent en une quantité allant par exemple de 0,01 à 0,5 % du poids total de la composition et de préférence de 0,01 à 0,05 % du poids total de la composition.

La composition selon l'invention comprend au moins une phase aqueuse et une phase huileuse distinctes.

La phase aqueuse de la composition de l'invention peut comprendre de l'eau déminéralisée stérile et/ou une eau florale telle que de l'eau de rose, de l'eau de bleuet, de l'eau de camomille ou de l'eau de tilleul, ou une eau thermale ou minérale naturelle, comme par exemple: l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La phase huileuse de la composition selon l'invention comprend au moins une huile, celle-ci pouvant être choisie parmi les huiles minérales, végétales ou synthétiques ou encore les huiles de silicone, et leurs mélanges.

Parmi les huiles minérales pouvant constituer la phase huileuse, on peut notamment citer l'huile de vaseline et les hydrocarbures aliphatiques supérieurs tels que par exemple l'isohexadécane ou l'isododécane ; parmi les huiles végétales, l'huile de jojoba, ainsi que l'huile de carthame ; parmi les huiles de silicone éventuellement volatiles, les cyclométhicones telles que le cyclopentadiméthylsiloxane, et parmi les huiles de synthèse, les palmitates d'alkyle où le groupe alkyle a de 2 à 10 atomes de carbone, tels que le palmitate d'isopropyle ou le palmitate d'octyle, et les adipates d'alkyle où le groupe alkyle a de 2 à 10 atomes de carbone, tels que l'adipate de di-éthyl-2-hexyle, ou tout autre ester aliphatique comportant de 12 à 20 atomes de carbone, et leurs mélanges.

Selon une forme de réalisation particulière de l'invention, la phase huileuse contient au moins au moins une huile choisie parmi les palmitates d'alkyle, l'isohexadécane, l'isododécane, les huiles de silicone volatiles (cyclométhicones) et leurs mélanges.

Quand la composition contient un palmitate d'alkyle, ce dernier est de préférence présent en une proportion d'au moins 5 % et mieux en une proportion allant de 8 à 30 % du poids total de la composition.

Quand la composition contient une huile de silicone volatile, la proportion de cette huile va par exemple de 5 à 50 % du poids total de la composition.

Selon une forme préférée de réalisation de l'invention, la phase huileuse contient au moins de l'isohexadécane ou de l'isododécane ou un mélange de ces deux huiles, en une proportion allant de 5 à 50 % et mieux 10 à 40 % du poids total de la composition.

La composition biphasée selon l'invention peut être exempte de tensioactif. Toutefois, elle peut comprendre aussi au moins un tensioactif dans l'une ou l'autre des phases.

Le tensioactif est de préférence du type anionique, non-ionique, ou amphotère. Il est généralement du type non-ionique. Il est de préférence présent dans la phase aqueuse. Il est de préférence présent en une proportion allant de 0,01 à 10 % (de matière active) en poids du poids total de la composition, et encore plus préférentiellement de 0,025 à 3 % du poids total de la composition.

Parmi les tensioactifs non-ioniques, ceux particulièrement préférés sont :
- les esters gras de sorbitol polyoxyéthylénés tels que le produit vendu sous la dénomination de TWEEN 20^{R} par la Société ATLAS.
- les alcools gras polyoxyéthylénés tels que le produit vendu sous la dénomination de REMCOPAL 21912 AL^{R} par la Société GERLAND.
- les alkylphénols polyoxyéthylénés tels que le produit vendu sous la dénomination de TRITON X 100^{R} par la Société ROHM-HAAS, et
- les condensats d'oxyde d'éthylène et d'oxyde de propylène (nom CTFA : Poloxamer) tels que ceux vendus sous les dénominations de SYNPERONIC PE^{R} par la Société ICI et en particulier ceux référencés L 31, L 64, F 38, F 88, L 92, P 103, F 108 et F 127.

Parmi les tensioactifs anioniques, on peut notamment citer :
- les alkyléthers sulfates tels que le produit vendu sous la dénomination de TEXAPON ASV^{R} par la Société HENKEL,
- les alkylsulfoacétates tels que produits vendu sous la dénomination de LATHANOL LAL^{R} par la Société STEPAN,
- les sulfosuccinates d'alkyle tels que le produit vendu sous la dénomination de SODIUM DIOCTYL SULFOSUCCINATE^{R} par la Société RHONE POULENC,
- les alkylamido sulfosuccinates tels que le produit vendu sous la dénomination de REWODERM S 1333^{R} par la Société REWO,
- les alkylamido polypeptides tels que le produit vendu sous la dénomination de LAMEPON S^{R} par la Société GRUNAU, et
- les acylsarcosinates tels que le produit vendu sous la dénomination de ORAMIX L 30^{R} par la Société SEPPIC.

Parmi les tensioactifs amphotères, on peut notamment citer :
- les alkylamidopropyl diméthylbétaïnes tels que le produit vendu sous la dénomination de TEGO BETAINE L 7^{R} par la Société GOLDSCHMIDT,
- les alkylamidobétaïnes tels que le produit vendu sous la dénomination de INCRONAM 30^{R} par la Société CRODA,
- les dérivés d'imidazoline tels que le produit vendu sous la dénomination de CHIMEXANE HD^{R} par la Société CHIMEX, et
- les N-alkyl-β-imino-dipropionates tels que le produit vendu sous la dénomination de MONATERIC ISA 35^{R} par la Société MONA.

De préférence, le rapport pondéral entre la phase aqueuse et la phase huileuse va de 30/70 à 60/40.

La composition de l'invention contient de manière appropriée un milieu physiologiquement acceptable, c'est-à-dire compatible avec la peau, les muqueuses et/ou les cheveux.

Outre les composés indiqués ci-dessus, la composition selon l'invention peut contenir des adjuvants cosmétiques conventionnels qui se trouveront dans l'une ou l'autre phase selon leur nature hydrophile ou lipophile, tels que par exemple des parfums, des colorants, des agents adoucissants, un tampon, des humectants, et éventuellement un électrolyte tel que le chlorure de sodium pour apporter une isotonicité dans la phase aqueuse ou tout autre composé approprié compatible avec la composition biphasée de l'invention.

Parmi les agents humectants, on peut notamment mentionner la glycérine, l'hexylèneglycol et le polyéthylèneglycol 600, ceux-ci étant présents à une concentration inférieure ou égale à 5% et de préférence allant de 0,05 à 2 % du poids total de la composition.

Parmi les agents adoucissants, on peut en particulier citer l'allantoïne, et certains extraits de plantes.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233 indiqués ici pour référence.

Avantageusement, l'invention concerne des compositions pour le soin et en particulier pour le nettoyage et/ou le démaquillage de la peau, des muqueuses telles que les lèvres, et/ou des yeux. Elle convient particulièrement pour le démaquillage des yeux dont les cils comportent du mascara, notamment pour le démaquillage des yeux sensibles, et pour le démaquillage des compositions de maquillage longue tenue et/ou dite "sans transfert" telles que décrites par exemple dans le document FR-A-2,747,566 indiqué ici comme pour référence.

Aussi, l'invention a encore pour l'objet un procédé cosmétique de nettoyage et/ou de démaquillage de la peau, des muqueuses et/ou des yeux, caractérisé en ce que l'on applique sur la peau, les muqueuses et/ou les yeux, une composition telle que définie ci-dessus.

L'invention a encore pour objet un procédé cosmétique et/ou dermatologique de démaquillage des yeux sensibles, consistant à appliquer sur les yeux une composition telle que définie ci-dessus.

On va maintenant donner à titre d'illustration, des exemples de compositions selon l'invention. Les pourcentages indiqués sont des pourcentages en poids.

### Exemple 1 : composition biphasique

- Palmitate d'octyle 9 %
- Isododécane 34 %
- Polyaminopropyl biguanide 0,03 %
- Poloxamer 184 (CTFA) 0,03 %
- Chlorure de sodium 0,5 %
- Hexylène glycol 1 %
- colorants qs
- Eau déminéralisée qsp 100 %

La composition obtenue se présente sous forme biphasée.

## Revendications

1. Composition cosmétique et/ou dermatologique biphasée constituée d'une phase aqueuse et d'une phase huileuse distinctes, **caractérisée en ce qu'**elle contient du chlorhydrate de poly-hexaméthylène biguanide.

2. Composition selon la revendication 1, **caractérisée en ce que** le chlorhydrate de poly-hexaméthylène biguanide est présent en une quantité allant de 0,01 à 0,5 % et de préférence de 0,01 à 0,05 % du poids total de la composition.

3. Composition selon la revendication 1 ou 2, **caractérisée en ce que en ce que** la phase huileuse contient au moins une huile choisie parmi les huiles de silicone, les hydrocarbures aliphatiques supérieurs et les huiles de synthèse.

4. Composition selon l'une quelconque des revendications précédentes, **caractérisée en ce que** la phase huileuse contient au moins une huile choisie parmi les palmitates d'alkyle, l'isohexadécane, l'isododécane, les huiles de silicone volatiles et leurs mélanges.

5. Composition selon l'une quelconque des revendications précédentes, **caractérisée en ce que** la phase huileuse contient au moins de l'isohexadécane ou de l'isododécane ou un mélange d'isohexadécane et d'isododécane, en une proportion allant de 5 à 50 % et de préférence 10 à 40 % du poids total de la composition.

6. Composition selon l'une quelconque des revendications précédentes, **caractérisée en ce qu'**elle contient au moins un tensioactif.

7. Composition selon la revendication 6, **caractérisée en ce que** le tensioactif est présent en une quantité allant de 0,01 à 10 % du poids total de la composition.

8. Composition selon l'une quelconque des revendications précédentes, **caractérisée en ce que** le rapport pondéral entre la phase aqueuse et la phase huileuse va de 30/70 à 60/40.

9. Procédé cosmétique de nettoyage et/ou de démaquillage de la peau, des muqueuses et/ou des yeux, **caractérisé en ce que** l'on applique sur la peau, les muqueuses et/ou les yeux, une composition telle que définie ci-dessus.

10. Procédé cosmétique de démaquillage des yeux sensibles, consistant à appliquer sur les yeux une composition selon l'une quelconque des revendications 1 à 8.

## Claims

1. Two-phase cosmetic and/or dermatological composition consisting of an aqueous phase and a separate oily phase, **characterized in that** it contains poly(hexamethylene biguanide) hydrochloride.

2. Composition according to Claim 1, **characterized in that** the poly(hexamethylene biguanide) hydrochloride is present in an amount ranging from 0.01 to 0.5% and preferably from 0.01 to 0.05% of the total weight of the composition.

3. Composition according to Claim 1 or 2, **characterized in that** the oily phase contains at least one oil chosen from silicone oils, higher aliphatic hydrocarbons and synthetic oils.

4. Composition according to any one of the preceding claims, **characterized in that** the oily phase contains at least one oil chosen from alkyl palmitates, isohexadecane, isododecane, volatile silicone oils and mixtures thereof.

5. Composition according to any one of the preceding claims, **characterized in that** the oily phase contains at least isohexadecane or isododecane or a mixture of isohexadecane and isododecane, in a proportion ranging from 5 to 50% and preferably 10 to 40% of the total weight of the composition.

6. Composition according to any one of the preceding claims, **characterized in that** it contains at least one surfactant.

7. Composition according to Claim 6, **characterized in that** the surfactant is present in an amount ranging from 0.01 to 10% of the total weight of the composition.

8. Composition according to any one of the preceding claims, **characterized in that** the weight ratio between the aqueous phase and the oily phase ranges from 30/70 to 60/40.

9. Cosmetic process for cleansing and/or removing make-up from the skin, mucous membranes and/or the eyes, **characterized in that** a composition as defined above is applied to the skin, mucous membranes and/or the eyes.

10. Cosmetic process for removing make-up from sensitive eyes, which consists in applying a composition according to any one of Claims 1 to 8 to the eyes.

## Patentansprüche

1. Zweiphasige kosmetische und/oder dermatologische Zusammensetzung, die aus einer wässrigen Phase und einer Ölphase besteht, welche voneinander verschieden sind, **dadurch gekennzeichnet, dass** sie das Polyhexamethylenbiguanid-Hydrochlorid enthält.

2. Zusammensetzung nach Anspruch 1, **dadurch gekennzeichnet, dass** das Polyhexamethylenbiguanid-Hydrochlorid in einer Menge von 0,01 bis 0,5 % und vorzugsweise 0,01 bis 0,05 % des Gesamtgewichts der Zusammensetzung vorliegt.

3. Zusammensetzung nach Anspruch 1 oder 2, **dadurch gekennzeichnet, dass** die Ölphase mindestens ein Öl enthält, das unter den Siliconölen, höheren aliphatischen Kohlenwasserstoffen und synthetischen Ölen ausgewählt ist.

4. Zusammensetzung nach einem der vorhergehenden Ansprüche, **dadurch gekennzeichnet, dass** die Ölphase mindestens ein Öl enthält, das unter den Alkylpalmitaten, Isohexadecan, Isododecan, flüchtigen Siliconölen und deren Gemischen ausgewählt ist.

5. Zusammensetzung nach einem der vorhergehenden Ansprüche, **dadurch gekennzeichnet, dass** die Ölphase zumindest Isohexadecan oder Isododecan oder ein Gemisch von Isohexadecan und Isododecan in einem Mengenanteil von 5 bis 50 % und vorzugsweise 10 bis 40 % des Gesamtgewichts der Zusammensetzung enthält.

6. Zusammensetzung nach einem der vorhergehenden Ansprüche, **dadurch gekennzeichnet, dass** sie mindestens einen grenzflächenaktiven Stoff enthält.

7. Zusammensetzung nach Anspruch 6, **dadurch gekennzeichnet, dass** der grenzflächenaktive Stoff in einer Menge von 0,01 bis 10 % des Gesamtgewichts der Zusammensetzung vorliegt.

8. Zusammensetzung nach einem der vorhergehenden Ansprüche, **dadurch gekennzeichnet, dass** das Gewichtsverhältnis von wässriger Phase und Ölphase im Bereich von 30/70 bis 60/40 liegt.

9. Kosmetisches Verfahren zur Reinigung und/oder zum Abschminken der Haut, der Schleimhäute und/oder der Augen, **dadurch gekennzeichnet, dass** auf die Haut, die Schleimhäute und/oder die Augen eine oben definierte Zusammensetzung aufgetragen wird.

10. Kosmetisches Verfahren zum Abschminken von empfindlichen Augen, **dadurch gekennzeichnet, dass** auf die Augen eine Zusammensetzung nach einem der Ansprüche 1 bis 8 aufgetragen wird.
